# EUROPÄISCHE PATENTSCHRIFT

(11) **EP 1 206 948 B1**
(45) Veröffentlichungstag und Bekanntmachung des Hinweises auf die Patenterteilung: **26.01.2005**
(21) Anmeldenummer: 01126358.9
(22) Anmeldetag: 07.11.2001
(51) Int. Cl.: A61M 16/00, A61M 15/00, G01F 1/00, A61B 5/00, G01N 1/00

(54) **Vorrichtung zur Entnahme von Gas aus der Atemluft bzw. Zufuhr von Gas zur Atemluft**
Apparatus for withdrawal of gas from the breathing air and supply of gas to the breathing air respectively
Appareil de prélèvement d'un gaz d'air respiré resp. d'alimentation d'un gaz à respirer

(30) Priorität: 17.11.2000 DE 10057040
(43) Veröffentlichungstag der Anmeldung: 22.05.2002
(73) Patentinhaber: Ganshorn, Peter, Dipl.-Ing., 97618 Niederlauer (DE)
(72) Erfinder: Ganshorn, Peter, Dipl.-Ing., 97618 Niederlauer (DE)
(74) Vertreter: Pöhner, Wilfried Anton, Dr.

(56) Entgegenhaltungen:
- EP-A- 0 520 201
- US-A- 5 666 945
- US-A- 5 842 468
- US-A- 6 029 660

## Beschreibung

Die Erfindung bezieht sich auf eine Vorrichtung zur Entnahme von Gas aus der Atemluft bzw. zur Zufuhr von Gas zur Atemluft mit einem Atemrohr, einem darin integrierten Durchflussmesser und einem an das Atemrohr angeschlossenen Behälter für Gase mit einer Steuereinheit.

Die Leistungsfähigkeit einer Lunge wird bestimmt durch die Effektivität beim Gasaustausch, d.h. bei der Abgabe von Sauerstoff an das Blut bzw. der Aufnahme von Kohlendioxid aus dem Blut. Beide Vorgänge sind von einer Vielzahl von Parametern abhängig, wie z.B. dem Lungenvolumen, der Verteilung der Atemluft innerhalb der Lunge, der Oberflächengröße der alveolokapillaren Membran, deren Stärke und Diffusionseigenschaften, oder der Ventilation. In all diesen Parametern können, bei einer Abweichung der Parameter von der Norm, auch Ursachen für Störungen der Lungentätigkeit begründet sein.

Zur Untersuchung dieser Störungen ist es üblich, die Ausatemluft auf deren Zusammensetzung hin zu analysieren, oder der Einatemluft bestimmte Zusatzgase zuzusetzen und dann deren Konzentration in der Ausatemluft zu überprüfen. Nach dem Stand der Technik werden hierfür Geräte eingesetzt, bei denen der Patient die Ausatemluft durch ein Atemrohr, meist mit eingebautem Durchflussmesser, an einen angeschlossenen Behälter abgibt. Die hierin aufgefangene Atemluft entstammt einem oder mehreren Ausatemzyklen. Die Analyse der Atemluft gibt daher auch nur über einen oder mehrere Zyklen gemittelte Meßwerte wieder.

Von besonderem medizinischen Interesse sind jedoch auch die Vorgänge beim Ein- und Ausatmen, die in einem kleinen Zeitabschnitt zwischen dem Beginn und dem Ende eines Ein- oder Ausatemzyklus ablaufen. Eine Analyse dieser Vorgänge ermöglicht eine sehr differenzierte Ursachenerforschung bei Lungenkrankheiten und sehr genaue Therapie bei Lungenfunktionsstörungen.Die o.g. Geräte sind hierfür jedoch ungeeignet, da sie keine auf kleine Zeitintervalle beschränkte Entnahmen von Gas aus der Ausatemluft zulassen.

Die für diesen Anwendungsfall eingesetzten Geräte beruhen in der Regel auf Anwendung der Massenspektrometrie und erfordern einen sehr hohen apparativen Aufwand. Geräte dieser Art stehen daher nur an großen Forschungsinstituten zur Verfügung, ein Einsatz bei Routineuntersuchungen oder in Arztpraxen scheidet aus Kostengründen aus.

Aus der gattungsbildenden US 6,029,660 ist ein Beatmungsgerät bekannt, bei dem die Atemluft dem Probanden unter Druck zugeführt wird und eine zusätzlichen Gaskomponenten beim Einatmen additiv überlagert wird. Diagnostische Untersuchungen sind nicht vorgesehen.

Vor diesem Hintergrund hat sich die Erfindung die Aufgabe gestellt, eine Vorrichtung zur Entnahme von Gas aus der Atemluft und zur Zufuhr von Gas zur Atemluft zu schaffen, die eine Analyse der Vorgänge zwischen dem Beginn und Ende eines Atemzyklus mit einfachen Mitteln erlaubt und daher für eine breite Anwendung geeignet ist.

Gelöst wird diese Aufgabe erfindungsgemäß durch die Vorrichtung wie sie in Anspruch 1 definiert ist.

Die vorgeschlagene Vorrichtung weist ein Atemrohr auf, durch dessen eines Ende hindurch der Patient ein- und ausatmet und dessen anderes Ende in der Raumluft endet. Das Aus- und Einatmen erfolgt daher in Abweichung zum Stand der Technik nicht in einen geschlossenen Behälter hinein oder aus einem geschlossenen Behälter heraus, sondern stets in einer Verbindung zur Raumluft. An dem Atemrohr sind wesentliche Elemente der Vorrichtung, ein Durchflussmesser und ein als Zylinder-Kolben-Anordnung ausgebildeter Behälter, angeschlossen. Der Anschluss des Zylinders ist so ausgeführt, dass Gas aus dem Atemrohr in den Zylinder und umgekehrt aus dem Zylinder in das Atemrohr fließen kann.

Der Kolben der Anordnung wird durch einen Servomotor angetrieben. Zur Ansteuerung des Servomotors ist eine Steuereinheit vorgesehen, welche die vom Durchflussmesser ermittelten Werte entgegennimmt und bei der Erzeugung der Signale für den Servomotor umsetzt. Nach einem Kerngedanken der Erfindung wird der Servomotor so angesteuert, dass die Geschwindigkeit des Kolbens proportional zum gemessenen Durchsatz im Atemrohr ist. Diese Ausgestaltung eröffnet, zusammen mit zusätzlichen Weiterbildungen der Erfindung, zahlreiche Anwendungsmöglichkeiten der erfindungsgemäßen Vorrichtung zu einer differenzierten Untersuchung der Lungentätigkeit.

Aus der Proportionalität der Kolbengeschwindigkeit zum Durchsatz im Atemrohr folgt, dass das vom Kolben pro Zeiteinheit geförderte Volumen zu jedem Zeitpunkt proportional zur Strömungsgeschwindigkeit im Atemrohr ist. Da die Geschwindigkeit der Luft im Atemrohr beim Ein- und Ausatmen zwischen Null und einem Maximalwert schwankt, unterliegt auch die Geschwindigkeit des Kolbens und damit das pro Zeiteinheit geförderte Volumen den gleichen Schwankungen. Dies bedeutet, dass bei schnellem Atemvorgang das vom Kolben pro Sekunde geförderte Volumen groß ist, bei Atempause gleich Null ist und beim Übergang vom Aus- zum Einatmen der Kolben seine Bewegungsrichtung ändert.

Bei Anwendung auf den Ausatemvorgang wird der Servomotor so angesteuert, dass der Kolben einen Ansaugtakt durchführt. Dabei wird über die Verbindung des Zylinders mit dem Atemrohr ein Teil der ins Freie strömenden Ausatemluft entnommen. Die pro Zeiteinheit entnommenen Menge ist dabei in jedem Zeitpunkt proportional zu der pro Zeiteinheit ausgeatmeten Menge, d.h. dem Durchsatz im Atemrohr. Das bedeutet aber, dass der Kolben in jedem Zeitpunkt während der Gasentnahme von der pro Zeiteinheit ausgeatmeten Menge den gleichen Bruchteil entnimmt. Damit entspricht auch die insgesamt entnommene Menge dem gleichen Bruchteil der insgesamt während der Dauer der Gasentnahme ausgeatmeten Menge.

Für einen Einatemvorgang gelten analoge Aussagen. Während des Einatmens führt der Kolben einen Ausstoßtakt durch, der dazu genutzt werden kann über die Verbindung des Zylinders mit dem Atemrohr der Einatemluft für Analysezwecke ein Zusatzgas zuzuführen. Dabei ist die pro Zeiteinheit zugeführte Menge in jedem Zeitpunkt proportional der in das Atemrohr pro Zeiteinheit einströmenden Menge der Einatemluft. Das bedeutet aber, dass in jedem Zeitpunkt während der Gaszuführung das zugeführte Gas in gleichbleibender Konzentration der Atemluft beigemengt wird. Die Atemluft muss dabei nicht, wie beim Stand der Technik, aus einem abgeschlossenen Gefäß entnommen werden, in dem vorab die Gasmischung hergestellt wurde, sondern kann kontinuierlich aus der Rumluft aufgenommen werden.

Ein ganz wesentlicher Vorteil der erfindungsgemäßen Vorrichtung kommt bei einer Weiterbildung zum Tragen. Diese Ausbildung der Vorrichtung weist eine Steuereinheit auf, durch welche Start- und Endzeitpunkt des Kolbenhubs bezüglich des Ein- oder Ausatembeginns vorgebbar sind. Die Vorgabe kann dabei beliebige Werte umfassen. Von besonderem Interesse sind gemäß einem Merkmal der Erfindung Start- und Endzeiten der Kolbenbewegung, welche
- einen Ausschnitt des Einatmen- oder Ausatemzyklus
- oder genau einen Einatmen- oder Ausatemzyklus
- oder mehrere Einatmen- oder Ausatemzyklen erfassen.

Die vorgeschlagene Vorrichtung ermöglicht damit auch den Zugang zu vorrangigen medizinisch Fragenstellungen, die sich auf die Vorgänge beim Einund Ausatmen, die in einem kleinen Zeitabschnitt zwischen dem Beginn und dem Ende eines Ein- oder Ausatemzyklus ablaufen, beziehen.

Betrachtet man beispielsweise einen Ausatemvorgang, lassen sich, durch Vorgabe der entsprechender Start- und Endzeiten, auf einfache Weise Gasentnahmen aus der Ausatemluft vornehmen, die beispielsweise kurz nach Beginn, im Maximum, oder vor Ende des Ausatemvorgangs liegen. Diese Gasentnahmen können ebenso genau einen Ein- oder Ausatemzyklus umfassen oder bei Bedarf auch die nachfolgenden Zyklen mit einschließen. Durch Analyse der entnommenen Gasvolumina ist es daher möglich, sowohl die Änderung in der Zusammensetzung der Atemluft über ein Atemintervall hinweg zu untersuchen, als auch die Entwicklung der Zusammensetzung von einem zum nächsten Atemintervall hin zu verfolgen.

Die Entnahme über mehrere Atemzyklen hinweg kann dabei so durchgeführt werden, dass jede entnommene Menge für sich analysiert wird oder jeweils im gleichen Zeitintervall entnommene Proben kumulativ über die Atemzyklen hinweg zu einem resultierenden Gasvolumen addiert und dann einer Analyse zugeführt werden.

In analoger Weise lassen sich beim Einatemvorgang in einem definierten Intervall, das vorzugsweise kleiner als die Einatemdauer gewählt wird, Zusatzgase der Einatemluft beimischen. Dabei sind die Intervalle sowohl innerhalb des Einatemzyklus beliebig vorgebbar, als auch zu beliebigen Zeiten bei nachfolgenden Einatemzyklen wiederholbar. Die auf diese Weise eingeatmeten Gase können mit der erfindungsgemäßen Vorrichtung in einem der nachfolgenden Ausatemzyklen wieder nachgewiesen werden und aus der Nachweiszeit und der Nachweismenge Rückschlüsse auf die Lungentätigkeit gezogen werden.

Die erfindungsgemäße Vorrichtung eröffnet damit einen Weg für medizinische Forschungen in einem bisher verschlossenen Bereich, ebenso auch einen Weg für Routineüberprüfungen in der ärztlichen Praxis, die bisher nicht möglich waren. Von besonderem Interesse sind dabei Untersuchungen von definierten Zeitabschnitten innerhalb eines Ein- oder Ausatemzyklus und von Mittelwerten, die sich über einen oder mehrere Zyklen hinweg erstrecken. Die Vorrichtung gemäß vorliegender Erfindung ermöglicht für beide Zielrichtungen sehr präzise Messungen.

Bei einer Variante dieser Weiterbildung der erfindungsgemäßen Vorrichtung werden statt des Start- und Endzeitpunkt des Kolbenhubs dessen Startzeit bezüglich des Ein- oder Ausatembeginns und das vom Kolben geförderte Volumen durch die Steuereinheit vorgeben. Bei dieser Ausführungsform führt der Kolben über ein oder mehrere Ein- oder Ausatemzyklen hinweg jeweils nur einen Aus- oder Einlasstakt aus, so lange bis das geförderte Volumen der Vorgabe entspricht. Fragestellungen dieser Art sind u.a. interessant bei Diffusionskapazitätsmessungen der Lunge.

Die Entnahme von Gas aus dem Atemrohr wird durch einen Ansaugtakt des Kolbens und die Zufuhr von Gas durch einen Ausstoßtakt des Kolbens durchgeführt. Die Kolbenbewegung ist dabei jeweils nur während der Gasentnahme oder Zufuhr aktiviert. In den Zwischenzeiten steht der Kolben still, daher findet auch bei offener Verbindung zwischen Atemrohr und Zylinder in diesen Zeiten kein Gasaustausch statt.

Für weitergehende Untersuchungen ist es jedoch notwendig, dass der Kolben auch von der Atemtätigkeit unabhängige Bewegungen durchführen kann. Bei einer vorteilhaften Ausführungsform der erfindungsgemäßen Vorrichtung ist daher zwischen Zylinder und Atemrohr wenigsten ein Ventil vorgesehen ist, welches den Durchfluss zwischen beiden Elementen freigibt oder sperrt. Dabei sind folgende Ventilstellungen möglich:
offen beim Ausatmen und geschlossen beim Einatmen
offen beim Einatmen und geschlossen beim Ausatmen
offen beim Einatmen und offen beim Ausatmen
geschlossen beim Einatmen und geschlossen beim Ausatmen.

Dabei sind die beiden erstgenannten Einstellungen von Bedeutung, wenn mit vorliegender Vorrichtung Messungen über mehrere Atemzyklen hinweg durchgeführt werden sollen und dabei entweder nur die Ausatemvorgänge oder nur die Einatemvorgänge einbezogen sein sollen. Die letztgenannte Einstellung wird benötigt, wenn die mit dem Kolben angesaugte Ausatemluft einem an den Zylinder angeschlossenen Analysegerät zugeführt werden soll. Die gleiche Einstellung ist auch notwendig, wenn in den Zylinder von außen her ein Gas aufgenommen wird, das bei einem nachfolgenden Einatemzyklus der Einatemluft beigemengt werden soll. Die Ansteuerung des Ventile wird von der Steuereinheit durchgeführt und erfolgt in Abhängigkeit von der Bewegungsrichtung des Kolbens.

Die genannte Ventilsteuerung bringt den Vorteil mit sich, dass ein und dieselbe Vorrichtung gemäß vorliegender Erfindung sowohl zur Entnahme und anschließenden Analyse von Gas aus der Ausatemluft als auch für die Zufuhr von Gas zur Einatemluft genutzt werden kann. Des weiteren ergibt sich durch die Ventilsteuerung eine erhöhte Flexibilität und eine erhöhte Sicherheit bei der Durchführung der Untersuchungen. So lassen sich beispielsweise Messungen über mehrere Ausatemzyklen hinweg in der Weise durchführen, dass der Kolben während der dazwischen liegenden Einatmemphasen einfach stillgesetzt wird. Zur Erhöhung der Sicherheit gegen unkontrollierten Gasaustausch ist es jedoch zweckmäßig, wenn während dieser Zeit gleichzeitig auch das Ventil zwischen Ausatemrohr und Zylinder geschlossen wird.

Zur Analyse der vom Kolben geförderten Ausatemluft ist bei einer Weiterbildung der Erfindung an den Zylinder der Vorrichtung ein Gerät zur Bestimmung der Gaszusammensetzung angeschlossen. Diese Analysen können Auskunft geben über Störungen der Lungentätigkeit.
Dabei sind zwei Kategorien von Untersuchungen üblich. Im ersten Fall wird die Ausatemluft selbst auf deren Zusammensetzung hin analysiert und im zweiten Fall der Einatemluft ein Zusatzgas zugesetzt und dann dessen Konzentration in der Ausatemluft ermittelt.

Der Zusatz von Gasen erfolgt dabei gemäß einem Merkmal der Erfindung über eine Leitung, die an das Atemrohr und / oder den Zylinder angeschlossen ist und ein Ventil aufweist, durch welches der Durchfluss freigebbar oder sperrbar ist.

Eine medizinische Fragestellung von besonderem Interesse betrifft die Untersuchung von Gasentnahmen, die in kurzen Zeitabständen und innerhalb einund desselben Atemzyklus durchgeführt werden. Durch Analyse dieser Gasproben erhält man Aussagen über die Veränderung der Gaszusammensetzung während eines Atemzyklus. Untersuchungen dieser Art sind bei einer bevorzugten Ausführungsform der erfindungsgemäßen Vorrichtung möglich. Sie zeichnet sich aus durch zwei oder mehrere Zylinder-Kolben-Anordnungen, die an das Atemrohr angeschlossen sind und je einen Servomotor zur Bewegung des Kolbens aufweisen. Durch die Steuereinheit werden bei Untersuchungen der genannten Art die einzelnen Kolben nach einander angesteuert und jeweils Start- und Endzeiten für die verschiedenen Kolben vorgegeben, so dass sie innerhalb eines Atemzyklus liegen. Durch jede Zylinder-Kolben-Anordnung wird dann eine Probe der Ausatemluft aus ein- und demselben Atemzyklus entnommen.

Weitere Vorteile, Merkmale und Einzelheiten der Erfindung lassen sich dem nachfolgenden Teil der Beschreibung entnehmen. In diesem Teil wird ein Ausführungsbeispiel der erfindungsgemäßen Vorrichtung anhand einer beigefügten Zeichnung naher erläutert.

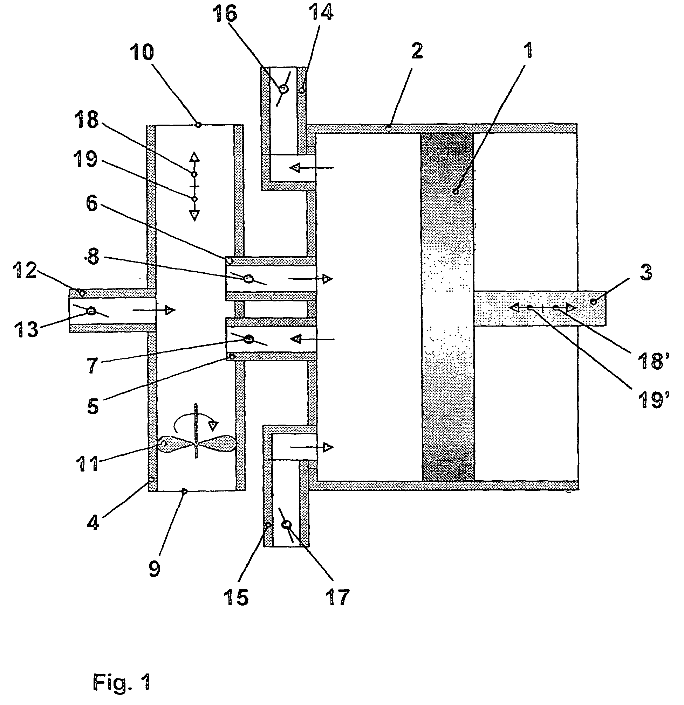

Die Figur 1 zeigt in vereinfachter Darstellung einen Schnitt durch die vorgeschlagene Vorrichtung.

In der Zeichnung ist der Kolben der Vorrichtung mit dem Bezugszeichen 1, der Zylinder mit dem Zeichen 2 wiedergegeben. Der Kolben ist über die Kolbenstange 3 mit einem nicht dargestellten Servomotor verbunden, während der Zylinder über zwei Verbindungsrohre mit dem Atemrohr 4 in Verbindung steht. Dabei wird das Verbindungsrohr 5 beim Einatmen, das Verbindungsrohr 6 beim Ausatmen genutzt. Die Verbindung durch beide Rohre kann mit je einem Ventil 7 bzw. 8 freigegeben oder gesperrt werden. Das Atemrohr führt von seinem einen Ende 9 aus zum Mund des Patienten, während das andere Ende 10 mit der Raumluft in Verbindung steht. Das Atemrohr weist einen Durchflussmesser auf, der mit dem Bezugszeichen 11 gekennzeichnet ist. Des weiteren ist an das Atemrohr eine Leitung 12 angeschlossen, über die Zusatzgase der Einatemluft beigemengt werden können. Auch diese Leitung kann mittels eines Ventils 13 abgesperrt werden. Am Zylinder sind ebenfalls weitere Leitungen für die Zufuhr und Abfuhr von Gasen vorgesehen. Die Leitung 14 dient der Weiterleitung der entnommenen Atemluft an ein nicht dargestelltes Analysegerät, die Leitung 15 der Beimischung von Zusatzgasen zur Einatemluft. Beide Leitungen können mittels je eines Ventil 16 bzw. 17 abgesperrt werden.

Mit dem Bezugszeichen 18 ist die Strömungsgeschwindigkeit im Atemrohr beim Ausatmen und mit 19 beim Einatmen angedeutet. Proportional zu diesen Geschwindigkeiten wird die Geschwindigkeit der Kolbenbewegung vorgegeben. Diese Abhängigkeit der Kolbengeschwindigkeit von der Stömungsgeschwindigkeit führt dazu, dass bei schnellem Atemvorgang das vom Kolben sekündlich geförderte Volumen groß ist, bei Atempause gleich Null ist und beim Übergang vom Aus- zum Einatmen der Kolben seine Bewegungsrichtung ändert. Beim Ausatmen des Patienten führt der Kolben einen Ansaugtakt durch, die Geschwindigkeit des Kolbens 18' weist dementsprechend vom Zylinderboden weg. Umgekehrt führt beim Einatmen des Patienten der Kolben einen Ausstoßtakt durch, die Geschwindigkeit des Kolbens 19' weist deshalb in Richtung zum Zylinderboden.

Durch die Vorgabe des Start- und Endzeitpunkt des Kolbenhubs durch die Steuereinheit lassen sich gezielte Entnahmen von Gas aus der Atemluft bzw. Zufuhren von Gas zur Atemluft durchführen. Von besonderem Interesse sind dabei Untersuchungen, die
- einen Ausschnitt des Einatmen- oder Ausatemzyklus
- oder genau einen Einatmen- oder Ausatemzyklus
- oder mehrere Einatmen- oder Ausatemzyklen
umfassen.

## Patentansprüche

1. Vorrichtung zur Entnahme von Gas aus der Atemluft bzw. Zufuhr von Gas zur Atemluft mit einem Atemrohr (4), einem darin integrierten Durchflussmesser (11) und einem an das Atemrohr (4) angeschlossenen Behälter für Gase, mit einer Steuereinheit, **dadurch gekennzeichnet, dass**
- das Atemrohr (4) so angeordnet ist, dass durch dessen eines Ende (9) hindurch der Patient ein- und ausatmen kann und dessen anderes Ende (10) in der Raumluft endet
- der Behälter als Zylinder-Kolben-Anordnung (1,2) ausgebildet ist, und dass der Anschluss des Zylinders (2) an des Atemrohr (4) so ausgeführt ist, dass Gas aus dem Atemrohr (4) in den Zylinder (2) und ungekehrt aus dem Zylinder (2) in das Atemrohr (4) fließen kann
- ein Servomotor zur Bewegung des Kolbens (1)
- und die Steuereinheit zur Ansteuerung des Servomotors vorgesehen ist
- und die Ansteuerung so erfolgt, dass die Geschwindigkeit des Kolbens proportional zu dem vom Durchflussmesser (11) gemessenen Durchsatz (Volumen pro Zeiteinheit) ist, sowie
- an dem Zylinder (2) eine Leitung (14, 15) angeschlossen ist, die ein Ventil (16,17) aufweist.

2. Vorrichtung zur Entnahme / Zufuhr von Gas nach Anspruch 1, **dadurch gekennzeichnet, dass** der Start- und Endzeitpunkt des Kolbenhubs bezüglich des Ein- oder Ausatembeginns durch die Steuereinheit vorgebbar ist.

3. Vorrichtung zur Entnahme / Zufuhr von Gas nach Anspruch 1, **dadurch gekennzeichnet, dass** der Startzeitpunkt des Kolbenhubs bezüglich des Einoder Ausatembeginns und das vom Kolben geförderte Volumen durch die Steuereinheit vorgebbar sind.

4. Vorrichtung zur Entnahme / Zufuhr von Gas nach einem der Ansprüche 1 - 3, **dadurch gekennzeichnet, dass** der Start- und Endzeitpunkt des Kolbenhubs einen Ausschnitt des Einatmen- oder Ausatemzyklus oder genau einen Einatmen- oder Ausatemzyklus oder mehrere Einatmen- oder Ausatemzyklen erfasst.

5. Vorrichtung zur Entnahme / Zufuhr von Gas nach einem der Ansprüche 1 - 4, **dadurch gekennzeichnet, dass** zwischen Zylinder (2) und Atemrohr (4) wenigstens ein Ventil (7,8) vorgesehen ist, welches den Durchfluss zwischen beiden Elementen freigibt oder sperrt.

6. Vorrichtung zur Entnahme / Zufuhr von Gas nach einem der Ansprüche 1 - 5, **dadurch gekennzeichnet, dass** an den Zylinder (2) ein Analysegerät zur Bestimmung der Zusammensetzung der vom Kolben (1) geförderten Ausatemluft angeschlossen ist.

7. Vorrichtung zur Entnahme / Zufuhr von Gas nach einem der Ansprüche 1 - 6, **dadurch gekennzeichnet, dass** an das Atemrohr (4) und/oder den Zylinder (2) je eine Leitung (12,15) für die Zufuhr von Gasen angeschlossen ist und die Leitung ein Ventil (13,17) aufweist, durch welches der Durchfluss freigebbar oder sperrbar ist.

8. Vorrichtung zur Entnahme / Zufuhr von Gas nach einem der Ansprüche 1 - 7, **dadurch gekennzeichnet, dass** wenigstens zwei Zylinder-Kolben-Anordnungen (1,2) an das Atemrohr (4) angeschlossen sind und je ein Servomotor zur Bewegung jedes Kolbens (1) vorgesehen ist.

## Claims

1. Device for removing a gas from the respiratory air or for feeding gas to the respiratory air, comprising a breathing tube (4), a flowmeter (11) integrated therein, and a container for gases, which is connected to the breathing tube (4), with a control unit, **characterised in that**
- the breathing tube (4) is arranged such that the patient can inhale and exhale through one end thereof, and its other end (10) terminates in the room air
- the container is designed as a cylinder-piston arrangement (1, 2), and **in that** the connection of the cylinder (2) to the breathing tube (4) is designed such that gas can flow out of the breathing tube (4) into the cylinder (2) and conversely out of the cylinder (2) into the breathing tube (4)
- a servo motor for moving the piston (1)
- and the control unit for actuating the servo motor is provided
- and actuation takes place such that the velocity of the piston is proportional to that of the flow (volume per unit time) measured by the flowmeter (11), and
- a line (14, 15) , which has a valve (16, 17) is connected to the cylinder (2).

2. Device for removing / feeding of gas according to claim 1, **characterised in that** the starting and ending time point of the piston stroke can be specified with reference to the beginning of inhalation or exhalation by means of the control unit.

3. Device for removing /feeding of gas according to claim 1, **characterised in that** the starting time point of the piston stroke with reference to the beginning of inhalation or exhalation and the volume delivered by the piston can be specified by means of the control unit.

4. Device for removing / feeding of gas according to one of claims 1 to 3, **characterized in that** the starting and ending time point of the piston stroke comprises a section of the inhalation or exhalation cycle or precisely one inhalation or exhalation cycle or a plurality of inhalation or exhalation cycles.

5. Device for removing /feeding of gas according to one of claims 1 to 4, **characterised in that** at least one valve (7, 8) is provided between the cylinder (2) and breathing tube (4) and releases or blocks the flow between the two elements.

6. Device for removing /feeding of gas according to one of claims 1 to 5, **characterised in that** an analysis unit for determining the composition of the exhaled air delivered by the piston (1) is connected to the cylinder (2).

7. Device for removing /feeding of gas according to one of claims 1 to 6, **characterised in that** one line (12, 15) for the feeding of gases is connected in each case to the breathing tube (4) and/or the cylinder (2) and the line has a valve (13, 17), by means of which the flow can be released or blocked.

8. Device for removing /feeding of gas according to one of claims 1 to 7, **characteried in that** at least two cylinder-piston arrangements (1, 2) are connected to the breathing tube (4) and one servo motor in each case is provided for moving each piston (1).

## Revendications

1. Dispositif permettant de prélever des gaz de l'air inhalé ou d'ajouter des gaz à l'air inhalé, comprenant un tube (4) dans lequel le patient respire, un débitmètre (11) intégré dans ce tube, et un récipient pour les gaz, relié au tube (4) dans lequel le patient respire, ainsi qu'un module de commande, **caractérisé en ce que**
- le tube (4) est placé de telle sorte que le patient inspire et expire à l'une de ses extrémités (9) tandis que sa deuxième extrémité (10) débouche dans l'air ambiant,
- le récipient se présente sous la forme d'un système composé d'un cylindre et d'un piston (1,2), le cylindre (2) étant raccordé au tube (4) dans lequel le patient respire de telle sorte que le gaz puisse passer du tube (4) dans le cylindre (2) et inversement, du cylindre (2) dans le tube (4),
- un servomoteur assure le déplacement du piston (1),
- le servomoteur est piloté par un module de commande,
- le pilotage est réalisé de manière à ce que la vitesse du piston soit proportionnelle au débit (volume par unité de temps) mesuré par le débitmètre (11), et
- le cylindre (2) est raccordé à une conduite (14, 15) renfermant une vanne (16, 17).

2. Dispositif permettant de prélever des gaz de l'air inhalé ou d'ajouter des gaz à l'air inhalé, suivant la revendication 1, **caractérisé en ce que** l'utilisateur peut programmer les temps de début et de fin de la course du piston par rapport au début de la phase d'inspiration ou d'expiration, dans le module de commande.

3. Dispositif permettant de prélever des gaz de l'air inhalé ou d'ajouter des gaz à l'air inhalé, suivant la revendication 1, **caractérisé en ce que** l'utilisateur programme seulement le temps de début de la course du piston par rapport au début de la phase d'inspiration ou d'expiration, et le volume devant être déplacé par le piston, dans le module de commande.

4. Dispositif permettant de prélever des gaz de l'air inhalé ou d'ajouter des gaz à l'air inhalé, suivant l'une des revendications 1 à 3, **caractérisé en ce que** les temps de début et de fin de la course du piston couvrent seulement une partie du cycle d'inspiration ou d'expiration ou exactement un cycle d'inspiration et d'expiration, ou encore plusieurs cycles d'inspiration et d'expiration.

5. Dispositif permettant de prélever des gaz de l'air inhalé ou d'ajouter des gaz à l'air inhalé, suivant l'une des revendications 1 à 4, **caractérisé en ce que** le dispositif comprend au moins une vanne (7, 8) située entre le cylindre (2) et le tube (4) dans lequel le patient respire, qui libère ou obture le passage entre les deux éléments.

6. Dispositif permettant de prélever des gaz de l'air inhalé ou d'ajouter des gaz à l'air inhalé, suivant l'une des revendications 1 à 5, **caractérisé en ce que** le cylindre (2) est raccordé à un appareil d'analyse permettant de déterminer la composition de l'air expiré, aspiré par le piston (1).

7. Dispositif permettant de prélever des gaz de l'air inhalé ou d'ajouter des gaz à l'air inhalé, suivant l'une des revendications 1 à 6, **caractérisé en ce que** le tube (4) dans lequel le patient respire et/ou le cylindre (2) sont respectivement reliés à une conduite (12, 15) permettant d'amener et d'évacuer les gaz, la conduite comprenant une vanne (13,17) qui obture ou libère le passage.

8. Dispositif permettant de prélever des gaz de l'air inhalé ou d'ajouter des gaz à l'air inhalé, suivant l'une des revendications 1 à 7, **caractérisé en ce que** le tube (4) dans lequel le patient respire est relié à au moins deux systèmes cylindre/piston (1, 2), chaque piston (1) étant déplacé par un servomoteur respectif.
